# FASCICULE DE BREVET EUROPEEN

(11) **EP 1 927 377 B1**
(45) Date de publication et mention de la délivrance du brevet: **26.12.2012**
(21) Numéro de dépôt: 07121666.7
(22) Date de dépôt: 27.11.2007
(51) Int. Cl.: A61Q 5/10, A61K 8/49

(54) **Composition de coloration de pH acide comprenant la 2,3-diamino-6,7-dihydro-1H,5H-pyrazolo[1,2-a]pyrazol-1-one, une para-phénylènediamine, un méta-aminophénol et un agent oxydant**
Saure Färbezusammensetzung enthaltend 2,3-Diamino-6,7-dihydro-1H,5H-pyrazolo[1,2-a]pyrazol-1-on, para-Phenylendiamin, meta-Aminophenol und Oxidationsmittel
Dye composition of acidic pH comprising 2,3-diamino-6,7-dihydro-1H,5H-pyrazolo[1,2-a]pyrazol-1-one, a para- phenylenediamine, a meta-aminophenol and an oxidizing agent

(30) Priorité: 30.11.2006 FR 0655214
(43) Date de publication de la demande: 04.06.2008
(73) Titulaire: L'Oréal, 75008 Paris (FR)
(72) Inventeur: Cottard, François, 92400, COURBEVOIE (FR); Laurent, Florence, 92270, BOIS COLOMBES (FR)
(74) Mandataire: Prevel, Estelle Nicole

(56) Documents cités:
- EP-A- 1 733 713
- EP-A- 1 733 714
- EP-A- 1 733 716
- US-A1- 2005 166 335

## Description

L'invention a pour objet une composition pour la teinture des fibres kératiniques, et en particulier des fibres kératiniques humaines telles que les cheveux, comprenant la 2,3-diamino-6,7-dihydro-1H,5H-pyrazolo[1,2-a]pyrazol-1-one ou l'un de ses sels d'addition à titre de première base d'oxydation, au moins une para-phénylènediamine à titre de deuxième base d'oxydation, au moins un méta-aminophénol à titre de coupleur et au moins un agent oxydant, le pH de la composition allant de 5,5 à 7,5.

Il est connu de teindre les fibres kératiniques, et en particulier les fibres kératiniques humaines telles que les cheveux, avec des compositions tinctoriales comprenant des précurseurs de colorant d'oxydation, en particulier des ortho ou para-phénylènediamines, des ortho ou para-aminophénols, des composés hétérocycliques tels que des dérivés de diaminopyrazole, des dérivés de pyrazolo[1,5-a]pyrimidine, des dérivés de pyrimidine, des dérivés de pyridine, des dérivés d'indole, des dérivés d'indoline, appelés généralement bases d'oxydation. Les précurseurs de colorants d'oxydation, ou bases d'oxydation, sont des composés incolores ou faiblement colorés qui, associés à des produits oxydants, peuvent donner naissance par un processus de condensation oxydative à des composés colorés ou colorants. On obtient ainsi des colorations permanentes.

On sait également que l'on peut faire varier les nuances obtenues avec ces bases d'oxydation en les associant à des coupleurs ou modificateurs de coloration, ces derniers étant choisis notamment parmi les méta-phénylènediamines, les méta-aminophénols, les méta-hydroxyphénols et certains composés hétérocycliques.

La variété des molécules mises en jeu au niveau des bases d'oxydation et des coupleurs permet l'obtention d'une riche palette de couleurs.

L'utilisation de bases d'oxydation classiques telles que le para-aminophénol, l'orthoaminophénol et leurs dérivés éventuellement associées à des coupleurs classiques à pH acide ne permettent pas d'obtenir des nuances reflets dans les tons rouge, cuivré ou acajou qui soient suffisamment visibles sur cheveux naturels ou colorés et homogènes de la racine à la pointe.

Le document US 2005/166335 A1 décrit une composition tinctoriale des fibres kératiniques comprenant, à titre de base d'oxydation, de la 2,3-diamino-6,7-dihydro-1H,5H-pyrazolo[1,2-a] pyrazol- 1-one, un coupleur choisi parmi les méta-aminophénols, un agent oxydant; le pH de la composition est 7.

Le but de la présente invention est de fournir de nouvelles compositions de coloration des fibres kératiniques qui permettent d'obtenir à pH acide une coloration aux reflets dans les tons rouge, cuivré ou acajou particulièrement visibles, puissante, chromatique, esthétique, peu sélective et résistant bien aux diverses agressions que peuvent subir les cheveux tels que les shampooings, la lumière, la sueur et les déformations permanentes.

La présente invention a donc pour objet une composition de coloration des fibres kératiniques comprenant, dans un milieu approprié pour la teinture :
- au moins une première base d'oxydation choisie parmi la 2,3-diamino-6,7-dihydro-1 H,5H-pyrazolo[1,2-a]pyrazol-1-one de formule (1) suivante et ses sels d'addition : 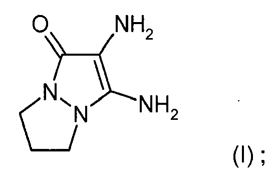
- au moins une deuxième base d'oxydation choisie parmi les para-phénylènediamines ;
- au moins un coupleur choisi parmi les méta-aminophénols ; et
- au moins un agent oxydant ;
   le pH de la composition allant de 5,5 à 7,5.

La présente invention permet d'obtenir une coloration des fibres kératiniques aux reflets dans les tons rouge, cuivré ou acajou qui soient suffisamment visibles sur cheveux naturels ou colorés et homogènes de la racine à la pointe.

Un autre objet de l'invention est un procédé de teinture des fibres kératiniques, et en particulier des fibres kératiniques humaines telles que les cheveux, mettant en oeuvre la composition de la présente invention, ainsi que l'utilisation de cette composition pour la teinture des fibres kératiniques.

L'invention a enfin pour objet un kit de coloration comprenant d'une part une composition de coloration contenant la 2,3-diamino-6,7-dihydro-1H,5H-pyrazolo[1,2-a]pyrazol-1-one ou l'un de ses sels d'addition à titre de première base d'oxydation, une para-phénylènediamine à titre de deuxième base d'oxydation et un méta-aminophénol à titre de coupleur et d'autre part une composition contenant un agent oxydant.

A moins d'une indication différente, les bornes des gammes de valeurs qui sont données dans le cadre de la présente invention sont incluses dans ces gammes.

Dans le cadre de l'invention, on entend par radical alkyle des radicaux alkyle linéaires ou ramifiés en C₁-C₁₀ sauf indication contraire, préférentiellement en C₁-C₆, encore plus préférentiellement en C₁-C₄, tels que le radical méthyle, éthyle, propyle, isopropyle, isobutyle, tertiobutyle, pentyle, hexyle.

Dans le cadre de la présente invention, le ou les hétéroatomes peuvent être choisis parmi un atome d'oxygène, un atome d'azote, un atome de soufre, un atome de phosphore.

Dans le cadre de la présente invention, un atome d'halogène peut être choisi parmi un atome de chlore, un atome de brome, un atome d'iode et un atome de fluor.

La ou les bases d'oxydation choisies parmi la 2,3-diamino-6,7-dihydro-1H,5H-pyrazolo[1,2-a]pyrazol-1-one de formule (I) et ses sels d'addition sont en général présentes dans la composition de l'invention chacune en quantité comprise entre 0,001 à 10 % en poids environ du poids total de la composition tinctoriale, de préférence entre 0,005 et 6 %.

Selon un mode de réalisation particulier de l'invention, la ou les para-phénylènediamines sont choisies parmi les composés de formule (II) suivante et leurs sels d'addition : 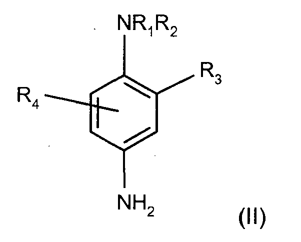 dans laquelle :
R₁ représente un atome d'hydrogène, un radical alkyle en C₁-C₄, monohydroxyalkyle en C₁-C₄, polyhydroxyalkyle en C₂-C₄, alcoxy(C₁-C₄)alkyle(C₁-C₄), alkyle en C₁-C₄ substitué par un groupement azoté, phényle ou 4'-aminophényle ;
R₂ représente un atome d'hydrogène, un radical alkyle en C₁-C₄, monohydroxyalkyle en C₁-C₄ ou polyhydroxyalkyle en C₂-C₄, alcoxy(C₁-C₄)alkyle(C₁-C₄) ou alkyle en C₁-C₄ substitué par un groupement azoté ;
R₁ et R₂ peuvent également former entre eux et avec l'atome d'azote auquel ils sont reliés un groupement cyclique contenant un ou plusieurs hétéroatomes, à 5 ou 6 chaînons, saturé ou insaturé, non substitué ou substitué par un ou plusieurs groupements alkyle, hydroxy ou uréido ;
R₃ représente un atome d'hydrogène, un atome d'halogène tel qu'un atome de chlore, un radical alkyle en C₁-C₄, sulfo, carboxy, monohydroxyalkyle en C₁-C₄ ou hydroxyalcoxy en C₁-C₄, acétylaminoalcoxy en C₁-C₄, mésylaminoalcoxy en C₁-C₄ ou carbamoylaminoalcoxy en C₁-C₄,
R₄ représente un atome d'hydrogène, d'halogène ou un radical alkyle en C₁-C₄.

Parmi les groupements azotés de la formule (II) ci-dessus, on peut citer notamment les radicaux amino, monoalkyl(C₁-C₄)amino, dialkyl(C₁-C₄)amino, trialkyl(C₁-C₄)amino, monohydroxyalkyl(C₁-C₄)amino, imidazolinium et ammonium.

Parmi les paraphénylènediamines de formule (II) ci-dessus, on peut plus particulièrement citer la paraphénylènediamine, la paratoluènediamine, la 2-chloro-paraphénylènediamine, la 2,3-diméthyl-paraphénylènediamine, la 2,6-diméthyl-paraphénylènediamine, la 2,6-diéthyl-paraphénylènediamine, la 2,5-diméthyl-paraphénylènediamine, la N,N-diméthyl-paraphénylènediamine, la N,N-diéthyl-paraphénylènediamine, la N,N-dipropyl-paraphénylènediamine, la 4-amino-N,N-diéthyl-3-méthyl-aniline, la N,N-bis-(β-hydroxyéthyl)-paraphénylènediamine, la 4-N,N-bis-(β-hydroxyéthyl)amino-2-méthyl-aniline, la 4-N,N-bis-(β-hydroxyéthyl)-amino 2-chloro-aniline, la 2-β-hydroxyéthyl-paraphénylènediamine, la 2-fluoro-paraphénylènediamine, la 2-isopropyl-paraphénylènediamine, la N-(β-hydroxypropyl)-paraphénylènediamine, la 2-hydroxyméthyl-paraphénylènediamine, la N,N-diméthyl-3-méthyl-paraphénylènediamine, la N,N-(éthyl,β-hydroxyéthyl)-paraphénylènediamine, la N-(β,γ-dihydroxypropyl)-paraphénylènediamine, la N-(4'-aminophényl)-paraphénylènediamine, la N-phényl-paraphénylènediamine, la 2-β-hydroxyéthyloxy-paraphénylènediamine, la 2-β-acétylaminoéthyloxy-paraphénylènediamine, la N-(β-méthoxyéthyl)-paraphénylènediamine, la 4-aminophénylpyrrolidine, lec 2-β-hydroxyéthylamino 5-amino toluène, la 2-méthyl-1-N-β-hydroxyéthyl-paraphénylènediamine, la 3-hydroxy 1-(4'-aminophényl)pyrrolidine, et leurs sels d'addition avec un acide.

Parmi les paraphénylènediamines de formule (II) ci-dessus, on préfère tout particulièrement la paraphénylènediamine, la paratoluènediamine, la 2-isopropyl-paraphénylènediamine, la 2-β-hydroxyéthyl-paraphénylènediamine, la 2-β-hydroxyéthyloxy-paraphénylènediamine, la 2,6-diméthyl-paraphénylène-diamine, la 2,6-diéthyl-paraphénylènediamine, la 2,3-diméthyl-paraphénylènediamine, la N,N-bis-(β-hydroxyéthyl)-paraphénylènediamine, la 2-chloro-paraphénylènediamine, la 2-β-acétylaminoéthyloxy-paraphénylènediamine, et leurs sels d'addition avec un acide.

Dans la composition de la présente invention, la ou les bases d'oxydation choisies parmi les para-phénylènediamines sont en général présentes chacune en quantité comprise entre 0,001 à 10 % en poids environ du poids total de la composition tinctoriale, de préférence entre 0,005 et 6 %.

Selon un mode de réalisation particulier de l'invention, le ou les méta-aminophénols sont choisis parmi les composés de formule (III) suivante et leurs sels d'addition : 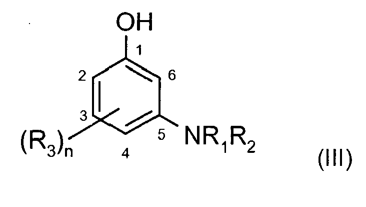 dans laquelle :
R₁ et R₂, identiques ou différents, représentent un atome d'hydrogène ; un radical alkyle ; un radical monohydroxyalkyle ; un radical polyhydroxyalkyle ; un radical monoaminoalkyle ;
R₁ et R₂ peuvent également former entre eux et avec l'atome d'azote auquel ils sont reliés un groupement cyclique contenant un ou plusieurs hétéroatomes, de 5 à 7 chaînons, saturé ou insaturé, non substitué ou substitué par un ou plusieurs radicaux choisis parmi les radicaux carboxy, carboxamido, hydroxyle, amino, mono ou dialkylamino, alkyle éventuellement substitué par un ou plusieurs radicaux hydroxyle, amino, mono ou dialkylamino ;
R₃ représentent, indépendamment les uns des autres, un atome d'halogène ; un radical alkyle ; un radical alcoxy ; un radical monohydroxyalkyle ; un radical polyhydroxyalkyle ; un radical monohydroxyalcoxy ; un radical polyhydroxyalcoxy ;
   n est un entier compris entre 0 et 3.

Selon un mode de réalisation particulier de l'invention, R₁ et R₂ représentent, indépendamment l'un de l'autre, un atome d'hydrogène ; un radical alkyle par exemple un radical méthyle ou éthyle ; un radical monohydroxyalkyle par exemple un radical β-hydroxyéthyle ou γ-hydroxypropyle ; ou R₁ et R₂ forment entre eux et avec l'atome d'azote auquel ils sont reliés un cycle choisi parmi les hétérocycles pyrrolidine, pipéridine, homopipéridine, pipérazine, homopipérazine, morpholine ; lesdits cycles pouvant être substitués par un ou plusieurs radicaux hydroxyle, amino, mono ou dialkyl(C₁-C₂)amino, carboxy, carboxamido, alkyle en C₁-C₄ éventuellement substitué par un ou plusieurs radicaux hydroxyle, amino, mono ou dialkyl(C₁-C₂)amino, et plus particulièrement choisi parmi la pyrrolidine, le 2,5-diméthylpyrrolidine, l'acide pyrrolidine-2-carboxylique, l'acide 3-hydroxypyrrolidine-2-carboxylique, l'acide 4-hydroxypyrrolidine-2-carboxylique, la 2,4-dicarboxypyrrolidine, la 3-hydroxy-2-hydroxyméthylpyrrolidine, la 2-carboxamidopyrrolidine, la 3-hydroxy-2-carboxamidopyrrolidine, la 2-hydroxyméthyl pyrrolidine, la 3,4-dihydroxy-2-hydroxyméthyl pyrrolidine, la 3-hydroxypyrrolidine, la 3,4-dihydroxy pyrrolidine, la 3-amino pyrrolidine, la 3-méthylamino pyrrolidine, la 3-diméthylamino-pyrrolidine, la 4-amino-3-hydroxy pyrrolidine, la 3-hydroxy-4-(β-hydroxyéthyl)amino-pyrrolidine, la pipéridine, la 2,6-diméthylpipéridine, la 2-carboxypipéridine, la 2-carboxamidopipéridine, la 2-hydroxyméthylpipéridine, la 3-hydroxy-2-hydroxyméthylpipéridine, la 3-hydroxypipéridine, la 4-hydroxypipéridine, la 3-hydroxyméthylpipéridine, la homopipéridine, la 2-carboxyhomopipéridine, la 2-carboxamidohomopipéridine, l'homopipérazine, le N-méthyl-homopipérazine, le N-(2-hydroxyéthyl)-homopipérazine, la pipérazine, la 4-méthyl-pipérazine, la 4-éthyl-pipérazine, la 4-(β-hydroxyéthyl)-pipérazine, la morpholine, et plus particulièrement ils forment un groupement pyrrolidin-1-yle ; pipéridin-1-yle ; pipérazin-1-yle ; 4-méthyl-pipérazin-1-yle ; 4-éthyl-pipérazin-1-yle ; 4-(β-hydroxyéthyl)-pipérazin-1-yle ; morpholin-4-yle.

Selon un mode de réalisation particulier de l'invention, R₃ est choisi parmi un atome d'halogène, un radical alkyle, un radical alcoxy, un radical monohydroxyalcoxy. A titre d'exemple, R₃ est choisi parmi un atome de chlore, un radical méthyle, un radical méthoxy, un radical β-hydroxyéthyloxy.

Selon un mode de réalisation particulier de l'invention, n désigne 0, 1 ou 2. A titre d'exemple, n est égal à 1 ou 2. Lorsque n est égal à 1, R₃ peut se situer en position 2 ou 6 et lorsque n est égal à 2, R₃ peuvent se situer en positions 2 et 4 ou en position 2 et 6.

Parmi les méta-aminophénols substitués de formule (III) utiles dans le cadre de l'invention, on peut plus particulièrement citer le 5-amino 2-méthoxy phénol, le 5-amino 2-(β-hydroxyéthyloxy) phénol, le 5-amino 2-méthyl phénol, le 5-N-(β-hydroxyéthyl)amino 2-méthyl phénol, le 5-N-(β-hydroxyéthyl)amino 4-méthoxy 2-méthyl phénol, le 5-amino 4-méthoxy 2-méthyl phénol, le 5-amino 4-chloro 2-méthyl phénol, le 6-chloro 2-méthyl 5-amino phénol, le 5-amino 2,4-diméthoxy phénol, le 5-(γ-hydroxypropylamino) 2-méthyl phénol, le 3-diméthylamino-phénol ; le 2-méthyl-5-diméthylamino-phénol ; le 2-éthyl-5-diméthylamino-phénol ; le 2-méthoxy-5-diméthylamino-phénol ; le 2-éthoxy-5-diméthylamino-phénol ; le 2-(β-hydroxyéthyl)-5-diméthylamino-phénol ; le 3-diéthylamino-phénol ; le 2-méthyl-5-diéthylamino-phénol ; le 2-éthyl-5-diéthylamino-phénol ; le 2-méthoxy-5-diéthylamino-phénol ; le 2-éthoxy-5-diéthylamino-phénol ; le 2-(β-hydroxyéthyl)-5-diéthylamino-phénol ; le 3-di(β-hydroxyéthyl)amino-phénol ; le 2-méthyl-5-di(β-hydroxyéthyl)amino-phénol ; le 2-éthyl-5-di(β-hydroxyéthyl)amino-phénol ; le 2-méthoxy-5-di(β-hydroxyéthyl)amino-phénol ; le 2-éthoxy-5-di(β-hydroxyéthyl)amino-phénol ; le 2-(β-hydroxyéthyl)-5-di(β-hydroxyéthyl)amino-phénol ; le 3-pyrrolidin-1-yl-phénol ; le 2-méthyl-5-pyrrolidin-1-yl-phénol ; le 2-éthyl-5-pyrrolidin-1-yl-phénol ; le 2-méthoxy-5-pyrrolidin-1-yl-phénol ; le 2-éthoxy-5-pyrrolidin-1-yl-phénol ; le 2-(β-hydroxyéthyl)-5-pyrrolidin-1-yl-phénol ; le 3-pipéridin-1-yl-phénol ; le 2-méthyl-5-pipéridin-1-yl-phénol ; le 2-éthyl-5-pipéridin-1-yl-phénol ; le 2-méthoxy-5-pipéridin-1-yl-phénol ; le 2-éthoxy-5-pipéridin-1-yl-phénol ; le 2-(β-hydroxyéthyl)-5-pipéridin-1-yl-phénol ; le 3-pipérazin-1-yl-phénol ; le 2-méthyl-5-pipérazin-1-yl-phénol ; le 2-éthyl-5-pipérazin-1-yl-phénol ; le 2-méthoxy-5-pipérazin-1-yl-phénol ; le 2-éthoxy-5-pipérazin-1-yl-phénol ; le 2-(β-hydroxyéthyl)-5-pipérazin-1-yl-phénol ; le 3-(4-méthyl-pipérazin-1-yl)-phénol ; le 2-méthyl-5-(4-méthyl-pipérazin-1-yl)-phénol ; le 2-éthyl-5-(4-méthyl-pipérazin-1-yl)-phénol ; le 2-méthoxy-5-(4-méthyl-pipérazin-1-yl)-phénol ; le 2-éthoxy-5-(4-méthyl-pipérazin-1-yl)-phénol ; le 2-(β-hydroxyéthyl)-5-(4-méthyl-pipérazin-1-yl)-phénol ; le 3-(4-éthyl-pipérazin-1-yl)-phénol ; le 2-méthyl-5-(4-éthyl-pipérazin-1-yl)-phénol ; le 2-éthyl-5-(4-éthyl-pipérazin-1-yl)-phénol ; le 2-méthoxy-5-(4-éthyl-pipérazin-1-yl)-phénol ; le 2-éthoxy-5-(4-éthyl-pipérazin-1-yl)-phénol ; le 2-(β-hydroxyéthyl)-5-(4-éthyl-pipérazin-1-yl)-phénol ; le 3-(4-(β-hydroxyéthyl)-pipérazin-1-yl)-phénol; le 2-méthyl-5-(4-(β-hydroxyéthyl)-pipérazin-1-yl)-phénol ; le 2-éthyl-5-(4-(β-hydroxyéthyl)-pipérazin-1-yl)-phénol ; le 2-méthoxy-5-(4-(β-hydroxyéthyl)-pipérazin-1-yl)-phénol ; le 2-éthoxy-5-(4-(β-hydroxyéthyl)-pipérazin-1-yl)-phénol ; le 2-(β-hydroxyéthyl)-5-(4-(β-hydroxyéthyl)-pipérazin-1-yl)-phénol ; le 3-morpholin-4-yl-phénol ; le 2-méthyl-5-morpholin-4-yl-phénol ; le 2-éthyl-5-morpholin-4-yl-phénol ; le 2-méthoxy-5-morpholin-4-yl-phénol ; le 2-éthoxy-5-morpholin-4-yl-phénol ; le 2-(β-hydroxyéthyl)-5-morpholin-4-yl-phénol.

Selon un mode de réalisation préféré, R₁ et R₂ désignent indépendemment l'un de l'autre un atome d'hydrogène ou un radical mono ou polyhydroxyalkyle. Le 5-amino 2-méthyl phénol et le 5-[N-(β-hydroxyéthyl)amino] 2-méthyl phénol sont particulièrement préférés.

Selon un autre mode de réalisation préféré, le ou les méta-aminophénols sont choisis parmi les méta-aminophénols chlorés. Dans le sens de la présente invention, on entend par méta-aminophénol chloré un méta-aminophénol comprenant dans sa structure au moins un atome de chlore. Le 6-chloro 2-méthyl 5-amino phénol est particulièrement préféré.

Dans la composition de la présente invention, le ou les coupleurs choisis parmi les méta-aminophénols sont en général présents chacun en quantité comprise entre 0,001 et 10 % en poids environ du poids total de la composition tinctoriale, de préférence entre 0,005 et 6 %.

La composition de la présente invention peut comprendre au moins une base d'oxydation additionnelle choisie parmi les bis-phénylalkylènediamines, les para-aminophénols, les bis-para-aminophénols, les ortho-aminophénols, les ortho-phénylènediamines, les bases hétérocycliques différentes de la 2,3-diamino-6,7-dihydro-1H,5H-pyrazolo[1,2-a]pyrazol-1-one et ses sels d'addition.

Parmi les bis-phénylalkylènediamines, on peut citer, à titre d'exemple, le N,N'-bis-(P-hydroxyéthyl) N,N'-bis-(4'-aminophényl) 1,3-diamino propanol, la N,N'-bis-(β-hydroxyéthyl) N,N'-bis-(4'-aminophényl) éthylènediamine, la N,N'-bis-(4-aminophényl) tétraméthylènediamine, la N,N'-bis-(β-hydroxyéthyl) N,N'-bis-(4-aminophényl) tétraméthylènediamine, la N,N'-bis-(4-méthyl-aminophényl) tétraméthylènediamine, la N,N'-bis-(éthyl) N,N'-bis-(4'-amino, 3'-méthylphényl) éthylènediamine, le 1,8-bis-(2,5-diamino phénoxy)-3,6-dioxaoctane, et leurs sels d'addition.

Parmi les para-aminophénols, on peut citer à titre d'exemple, le para-aminophénol, le 4-amino 3-méthyl phénol, le 4-amino 3-fluoro phénol, le 4-amino 3-hydroxyméthyl phénol, le 4-amino 2-méthyl phénol, le 4-amino 2-hydroxyméthyl phénol, le 4-amino 2-méthoxyméthyl phénol, le 4-amino 2-aminométhyl phénol, le 4-amino 2-(β-hydroxyéthyl aminométhyl) phénol, le 4-amino 2-fluoro phénol, et leurs sels d'addition avec un acide.

Parmi les ortho-aminophénols, on peut citer, à titre d'exemple, le 2-amino phénol, le 2-amino 5-méthyl phénol, le 2-amino 6-méthyl phénol, le 5-acétamido 2-amino phénol, et leurs sels d'addition.

Parmi les bases hétérocycliques, on peut citer, à titre d'exemple, les dérivés pyridiniques, les dérivés pyrimidiniques et les dérivés pyrazoliques.

Parmi les dérivés pyridiniques, on peut citer les composés décrits par exemple dans les brevets GB 1 026 978 et GB 1 153 196, comme la 2,5-diamino pyridine, la 2-(4-méthoxyphényl)amino 3-amino pyridine, la 2,3-diamino 6-méthoxy pyridine, la 2-(β-méthoxyéthyl)amino 3-amino 6-méthoxy pyridine, la 3,4-diamino pyridine, et leurs sels d'addition.

D'autres bases d'oxydation pyridiniques utiles dans la présente invention sont les bases d'oxydation 3-amino pyrazolo-[1,5-a]-pyridines ou leurs sels d'addition décrits par exemple dans la demande de brevet FR 2 801 308. A titre d'exemple, on peut citer la pyrazolo[1,5-a]pyridin-3-ylamine ; la 2-acétylamino pyrazolo-[1,5-a] pyridin-3-ylamine ; la 2-morpholin-4-yl-pyrazolo[1,5-a]pyridin-3-ylamine ; l'acide 3-amino-pyrazolo[1,5-a]pyridin-2-carboxylique ; la 2-méthoxy-pyrazolo[1,5-a]pyridine-3-ylamino ; le (3-amino-pyrazolo[1,5-a]pyridine-7-yl)-méthanol ; le 2-(3-amino-pyrazolo[1,5-a]pyridine-5-yl)-éthanol ; le 2-(3-amino-pyrazolo[1,5-a]pyridine-7-yl)-éthanol ; le (3-amino-pyrazolo[1,5-a]pyridine-2-yl)-méthanol ; la 3,6-diamino-pyrazolo[1,5-a]pyridine ; la 3,4-diamino-pyrazolo[1,5-a]pyridine ; la pyrazolo[1,5-a]pyridine-3,7-diamine ; la 7-morpholin-4-yl-pyrazolo[1,5-a]pyridin-3-ylamine ; la pyrazolo[1,5-a]pyridine-3,5-diamine ; la 5-morpholin-4-yl-pyrazolo[1,5-a]pyridin-3-ylamine ; le 2-[(3-amino-pyrazolo[1,5-a]pyridin-5-yl)-(2-hydroxyéthyl)-amino]-éthanol ; le 2-[(3-amino-pyrazolo[1,5-a]pyridin-7-yl)-(2-hydroxyéthyl)-amino]-éthanol ; le 3-amino-pyrazolo[1,5-a]pyridine-5-ol ; le 3-amino-pyrazolo[1,5-a]pyridine-4-ol ; le 3-amino-pyrazolo[1,5-a]pyridine-6-ol ; le 3-amino-pyrazolo[1,5-a]pyridine-7-ol ; ainsi que leurs d'addition.

Parmi les dérivés pyrimidiniques, on peut citer les composés décrits par exemple dans les brevets DE 23 59 399 ; JP 88-169571 ; JP 05-63124 ; EP 0 770 375 ou demande de brevet WO 96/15765 comme la 2,4,5,6-tétra-aminopyrimidine, la 4-hydroxy 2,5,6-triaminopyrimidine, la 2-hydroxy 4,5,6-triaminopyrimidine, la 2,4-dihydroxy 5,6-diaminopyrimidine, la 2,5,6-triaminopyrimidine, et les dérivés pyrazolo-pyrimidiniques tels ceux mentionnés dans la demande de brevet FR-A-2 750 048 et parmi lesquels on peut citer la pyrazolo-[1,5-a]-pyrimidine-3,7-diamine ; la 2,5-diméthyl pyrazolo-[1,5-a]-pyrimidine-3,7-diamine ; la pyrazolo-[1,5-a]-pyrimidine-3,5-diamine ; la 2,7-diméthyl pyrazolo-[1,5-a]-pyrimidine-3,5-diamine ; le 3-amino pyrazolo-[1,5-a]-pyrimidin-7-ol ; le 3-amino pyrazolo-[1,5-a]-pyrimidin-5-ol ; le 2-(3-amino pyrazolo-[1,5-a]-pyrimidin-7-ylamino)-éthanol, le 2-(7-amino pyrazolo-[1,5-a]-pyrimidin-3-ylamino)-éthanol, le 2-[(3-amino-pyrazolo[1,5-a]pyrimidin-7-yl)-(2-hydroxy-éthyl)-amino]-éthanol, le 2-[(7-amino-pyrazolo[1,5-a]pyrimidin-3-yl)-(2-hydroxy-éthyl)-amino]-éthanol, la 5,6-diméthyl pyrazolo-[1,5-a]-pyrimidine-3,7-diamine, la 2,6-diméthyl pyrazolo-[1,5-a]-pyrimidine-3,7-diamine, la 2, 5, N 7, N 7-tetraméthyl pyrazolo-[1,5-a]-pyrimidine-3,7-diamine, la 3-amino-5-méthyl-7-imidazolylpropylamino pyrazolo-[1,5-a]-pyrimidine et leurs sels d'addition et leurs formes tautomères, lorsqu'il existe un équilibre tautomérique.

Parmi les dérivés pyrazoliques, on peut citer les composés décrits dans les brevets DE 38 43 892, DE 41 33 957 et demandes de brevet WO 94/08969, WO 94/08970, FR-A-2 733 749 et DE 195 43 988 comme le 4,5-diamino 1-méthyl pyrazole, le 4,5-diamino 1-(β-hydroxyéthyl) pyrazole, le 3,4-diamino pyrazole, le 4,5-diamino 1-(4'-chlorobenzyl) pyrazole, le 4,5-diamino 1,3-diméthyl pyrazole, le 4,5-diamino 3-méthyl 1-phényl pyrazole, le 4,5-diamino 1-méthyl 3-phényl pyrazole, le 4-amino 1,3-diméthyl 5-hydrazino pyrazole, le 1-benzyl 4,5-diamino 3-méthyl pyrazole, le 4,5-diamino 3-tert-butyl 1-méthyl pyrazole, le 4,5-diamino 1-tert-butyl 3-méthyl pyrazole, le 4,5-diamino 1-(β-hydroxyéthyl) 3-méthyl pyrazole, le 4,5-diamino 1-éthyl 3-méthyl pyrazole, le 4,5-diamino 1-éthyl 3-(4'-méthoxyphényl) pyrazole, le 4,5-diamino 1-éthyl 3-hydroxyméthyl pyrazole, le 4,5-diamino 3-hydroxyméthyl 1-méthyl pyrazole, le 4,5-diamino 3-hydroxyméthyl 1-isopropyl pyrazole, le 4,5-diamino 3-méthyl 1-isopropyl pyrazole, le 4-amino 5-(2'-aminoéthyl)amino 1,3-diméthyl pyrazole, le 3,4,5-triamino pyrazole, le 1-méthyl 3,4,5-triamino pyrazole, le 3,5-diamino 1-méthyl 4-méthylamino pyrazole, le 3,5-diamino 4-(β-hydroxyéthyl)amino 1-méthyl pyrazole, et leurs sels d'addition.

Lorsque la composition de l'invention comprend une ou plusieurs bases d'oxydation additionnelles, celles-ci sont généralement présentes dans la composition de l'invention sont en général présentes chacune en quantité comprise entre 0,001 à 10 % en poids environ du poids total de la composition tinctoriale, de préférence entre 0,005 et 6 %.

La composition de la présente invention peut comprendre au moins un coupleur additionnel choisi parmi choisis parmi les méta-phénylènediamines, les méta-diphénols, les coupleurs naphtaléniques, les coupleurs hétérocycliques et leurs sels d'addition.

A titre d'exemples, on peut citer le 1,3-dihydroxy benzène, le 1,3-dihydroxy 2-méthyl benzène, le 4-chloro 1,3-dihydroxy benzène, le 2,4-diamino 1-(β-hydroxyéthyloxy) benzène, le 2-amino 4-(β-hydroxyéthylamino) 1-méthoxybenzène, le 1,3-diamino benzène, le 1,3-bis-(2,4-diaminophénoxy) propane, la 3-uréido aniline, le 3-uréido 1-diméthylamino benzène, le sésamol, le 1-β-hydroxyéthylamino-3,4-méthylènedioxybenzène, l'α-naphtol, le 2 méthyl-1-naphtol, le 6-hydroxy indole, le 4-hydroxy indole, le 4-hydroxy N-méthyl indole, la 2-amino-3-hydroxy pyridine, la 6-hydroxy benzomorpholine la 3,5-diamino-2,6-diméthoxypyridine, le 1-N-(β-hydroxyéthyl)amino-3,4-méthylène dioxybenzène, le 2,6-bis-(β-hydroxyéthylamino)toluène et leurs sels d'addition.

Lorsque la composition de l'invention comprend un ou plusieurs coupleurs additionnels, ceux-ci sont généralement présents dans la composition de l'invention chacun en quantité comprise entre 0,001 et 10 % en poids environ du poids total de la composition tinctoriale, de préférence entre 0,005 et 6 %.

D'une manière générale, les sels d'addition des bases d'oxydation et des coupleurs utilisables dans le cadre de l'invention sont notamment choisis parmi les sels d'addition avec un acide tels que les chlorhydrates, les bromhydrates, les sulfates, les citrates, les succinates, les tartrates, les lactates, les alkyl(C₁-C₄)sulfonates, les tosylates, les benzènesulfonates, les phosphates et les acétates et les sels d'addition avec une base telles que la soude, la potasse, l'ammoniaque, les amines ou les alcanolamines.

Le ou les agents oxydants peuvent être choisis parmi les agents oxydants classiquement utilisés pour la teinture d'oxydation des fibres kératiniques comme par exemple le peroxyde d'hydrogène, le peroxyde d'urée, les bromates de métaux alcalins, les persels tels que les perborates et persulfates, les peracides et les enzymes oxydases parmi lesquelles on peut citer les peroxydases, les oxydoréductases à 2 électrons telles que les uricases et les oxygénases à 4 électrons comme les laccases, les enzymes oxydases étant éventuellement en présence de leurs cofacteurs. Le peroxyde d'hydrogène est particulièrement préféré.

Le ou les agents oxydants sont généralement présents en quantité comprise entre 0,01 et 30 %, de préférence entre 0,1 et 20 % en poids par rapport au poids total de la composition.

Le pH de la composition tinctoriale conforme à l'invention va de 5,5 à 7,5, et de préférence de 5,7 à 6,9. Il peut être ajusté à la valeur désirée au moyen d'agents acidifiants ou alcalinisants habituellement utilisés en teinture des fibres kératiniques ou bien encore à l'aide de systèmes tampons classiques.

Parmi les agents acidifiants, on peut citer, à titre d'exemple, les acides minéraux ou organiques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.

Parmi les agents alcalinisants on peut citer, à titre d'exemple, l'ammoniaque, les carbonates alcalins, les alcanolamines telles que les mono-, di- et triéthanolamines ainsi que leurs dérivés, les hydroxydes de sodium ou de potassium et les composés de formule (IV) suivante : 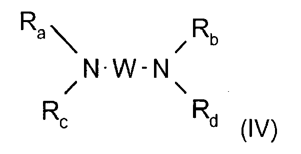 dans laquelle W est un reste propylène éventuellement substitué par un groupement hydroxyle ou un radical alkyle en C₁-C₄ ; Rₐ, R_{b}, R_{c} et R_{d}, identiques ou différents, représentent un atome d'hydrogène, un radical alkyle en C₁-C₄ ou hydroxyalkyle en C₁-C₄.

Le milieu approprié pour la teinture appelé aussi support de teinture est un milieu cosmétique généralement constitué par de l'eau ou par un mélange d'eau et d'au moins un solvant organique pour solubiliser les composés qui ne seraient pas suffisamment solubles dans l'eau. A titre de solvant organique, on peut par exemple citer les alcanols inférieurs en C₁-C₄, tels que l'éthanol et l'isopropanol ; les polyols et éthers de polyols comme le 2-butoxyéthanol, le propylèneglycol, le monométhyléther de propylèneglycol, le monoéthyléther et le monométhyléther du diéthylèneglycol, ainsi que les alcools aromatiques comme l'alcool benzylique ou le phénoxyéthanol, et leurs mélanges.

Les solvants sont, de préférence, présents dans des proportions de préférence comprises entre 1 et 40 % en poids environ par rapport au poids total de la composition tinctoriale, et encore plus préférentiellement entre 5 et 30 % en poids environ.

La composition tinctoriale conforme à l'invention peut également renfermer divers adjuvants utilisés classiquement dans les compositions pour la teinture des cheveux, tels que des agents tensio-actifs anioniques, cationiques, non-ioniques, amphotères, zwittérioniques ou leurs mélanges, des polymères anioniques, cationiques, non-ioniques, amphotères, zwittérioniques ou leurs mélanges, des agents épaississants minéraux ou organiques, et en particulier les épaississants associatifs polymériques anioniques, cationiques, non ioniques et amphotères, des agents antioxydants, des agents de pénétration, des agents séquestrants, des parfums, des tampons, des agents dispersants, des agents de conditionnement tels que par exemple des silicones volatiles ou non volatiles, modifiées ou non modifiées, des agents filmogènes, des céramides, des agents conservateurs, des agents opacifiants.

Les adjuvants ci dessus sont en général présents en quantité comprise pour chacun d'eux entre 0,01 et 20 % en poids par rapport au poids de la composition tinctoriale.

Bien entendu, l'homme de l'art veillera à choisir ce ou ces éventuels composés complémentaires de manière telle que les propriétés avantageuses attachées intrinsèquement à la composition de teinture d'oxydation conforme à l'invention ne soient pas, ou substantiellement pas, altérées par la ou les adjonctions envisagées.

La composition tinctoriale selon l'invention peut se présenter sous des formes diverses, telles que sous forme de liquides, de crèmes, de gels, ou sous toute autre forme appropriée pour réaliser une teinture des fibres kératiniques, et notamment des cheveux humains.

Le procédé conforme à la présente invention est un procédé dans lequel on applique sur les fibres la composition selon l'invention telle que définie précédemment pendant un temps suffisant pour développer la coloration désirée, après quoi on rince, on lave éventuellement au shampooing, on rince à nouveau et on sèche.

Le temps nécessaire au développement de la coloration sur les fibres kératiniques est généralement compris entre 2 et 60 minutes, de préférence entre 3 et 40 minutes, et encore plus préférentiellement entre 5 et 30 minutes.

Selon une forme de réalisation particulière de l'invention, le procédé comporte une étape préliminaire consistant à stocker sous forme séparée, d'une part, une composition (A) comprenant, dans un milieu approprié pour la teinture, au moins une première base d'oxydation choisie parmi la 2,3-diamino-6,7-dihydro-1H,5H-pyrazolo[1,2-a]pyrazol-1-one et ses sels d'addition, au moins une deuxième base d'oxydation choisie parmi les para-phénylènediamines et au moins un coupleur choisi parmi les méta-aminophénols et d'autre part, une composition (B) renfermant, dans un milieu approprié pour la teinture, au moins un agent oxydant, puis à procéder à leur mélange au moment de l'emploi avant d'appliquer ce mélange sur les fibres kératiniques.

Les compositions (A) et (B) peuvent également renfermer divers adjuvants utilisés classiquement dans les compositions pour la teinture des cheveux et tels que définis précédemment.

Le pH de la composition (A) est généralement compris entre 3 et 12 environ, et de préférence entre 5 et 11 environ. Il peut être ajusté à la valeur désirée au moyen d'agents acidifiants ou alcalinisants tels que définis précédemment ou bien encore à l'aide de systèmes tampons classiques.

Le pH de la composition (B) est tel qu'après mélange avec la composition (A), le pH de la composition résultante appliquée sur les fibres kératiniques va de 5,5 à 7,5, et encore plus préférentiellement de 5,6 à 6,9. Il peut être ajusté à la valeur désirée au moyen d'agents acidifiants ou alcalinisants tels que définis précédemment ou bien encore à l'aide de systèmes tampons classiques.

Un autre objet de l'invention est un dispositif à plusieurs compartiments ou "kit" de teinture ou tout autre système de conditionnement à plusieurs compartiments dont un premier compartiment renferme la composition (A) telle que définie ci-dessus et un second compartiment renferme la composition (B) telle que définie ci-dessus. Ces dispositifs peuvent être équipés d'un moyen permettant de délivrer sur les cheveux le mélange souhaité, tel que les dispositifs décrits dans le brevet FR-2 586 913 au nom de la demanderesse.

La présente invention a également pour objet l'utilisation pour la coloration d'oxydation des fibres kératiniques, et en particulier des fibres kératiniques humaines telles que les cheveux, d'une composition selon l'invention telle que définie précédemment.

Les exemples qui suivent servent à illustrer l'invention sans toutefois présenter un caractère limitatif.

### EXEMPLE

La composition suivante a été réalisée :

| | |
|---|---|
| Séquestrants | 2 g |
| Réducteurs | 0,71 g |
| Ethanolamine | 1,6 g |
| Acide citrique | 0,15 g |
| Silice pyrogénée à caractère hydrophobe | 1,2 g |
| 2,3-diamino-6,7-dihydro-1H,5H-pyrazolo[1,2-a]pyrazol-1-one, 2 CH₃-SO₃H | 1,5 g |
| 4-amino 2-hydroxy toluène | 1,1 g |
| 6-hydroxy indole | 0,2 g |
| Para-phénylènediamine | 0,4 g |
| Distéarate de glycol | 2 g |
| Parfum | 0,95 g |
| Polycondensat tétraméthyl hexaméthylènediamine / dichloro 1,3-propylène | 4 g |
| Copolymère chlorure de diméthyl diallyl ammonium / acide acrylique (80/20) | 3 g |
| Carbopol 980 | 0,4 g |
| Eau | 35,04 g |
| Propylène glycol | 10 g |
| Acide laurique | 3 g |
| Alcool laurique oxyéthyléné (12 OE) | 7 g |
| Alcool cétylstéarylique | 11,5 g |
| Alcool décylique oxyéthyléné (3 OE) | 10 g |
| Alcool oléocétylique oxyéthyléné (30 OE) | 4 g |
| Acide ascorbique | 0,25 g |

Au moment de l'emploi, 1 partie en poids de la composition décrite ci-dessus est mélangée avec 1 partie en poids d'une solution de peroxyde d'hydrogène à 20 volumes dont le pH est égal à 2,3. On obtient un pH final de 6,8 +/- 0,2.
Le mélange obtenu est appliqué sur des mèches de cheveux gris à 90 % de blancs naturels ou permanentés. Après 20 minutes de pose à 22 °C +/- 3 °C, les mèches sont rincées, lavées avec un shampooing standard, rincées à nouveau puis séchées.
La coloration capillaire est évaluée de manière visuelle. On obtient une nuance avec un intense reflet acajou.

## Revendications

1. Composition de coloration des fibres kératiniques comprenant, dans un milieu approprié pour la teinture :
• au moins une première base d'oxydation choisie parmi la 2,3-diamino-6,7-dihydro-1H,5H-pyrazolo[1,2-a]pyrazol-1-one de formule (I) suivante et ses sels d'addition : 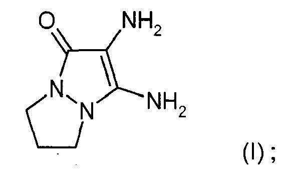
• au moins une deuxième base d'oxydation choisie parmi les para-phénylènediamines ;
• au moins un coupleur choisi parmi les méta-aminophénols ; et
• au moins un agent oxydant ;
le pH de la composition allant de 5,5 à 7,5.

2. Composition selon la revendication 1 dans laquelle la ou les bases d'oxydation choisies parmi la 2,3-diamino-6,7-dihydro-1H,5H-pyrazolo[1,2-a]pyrazol-1-one de formule (I) et ses sels d'addition sont présentes chacune en quantité comprise entre 0,001 à 10 % en poids environ du poids total de la composition tinctoriale.

3. Composition selon la revendication 1 ou 2 dans laquelle la ou les para-phénylènediamines sont choisies parmi les composés de formule (II) suivante et leurs sels d'addition : 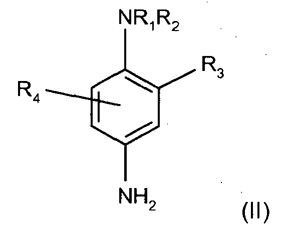 dans laquelle :
R₁ représente un atome d'hydrogène, un radical alkyle en C₁-C₄, monohydroxyalkyle en C₁-C₄, polyhydroxyalkyle en C₂-C₄, alcoxy(C₁-C4)alkyle(C₁-C₄), alkyle en C₁-C₄ substitué par un groupement azoté, phényle ou 4'-aminophényle ;
R₂ représente un atome d'hydrogène, un radical alkyle en C₁-C₄, monohydroxyalkyle en C₁-C₄ ou polyhydroxyalkyle en C₂-C₄, alcoxy(C₁-C₄)alkyle(C₁-C₄) ou alkyle en C₁-C₄ substitué par un groupement azoté ;
R₁ et R₂ peuvent également former entre eux et avec l'atome d'azote auquel ils sont reliés un groupement cyclique contenant un ou plusieurs hétéroatomes, à 5 ou 6 chaînons, saturé ou insaturé, non substitué ou substitué par un ou plusieurs groupements alkyle, hydroxy ou uréido ;
R₃ représente un atome d'hydrogène, un atome d'halogène, un radical alkyle en C₁-C₄, sulfo, carboxy, monohydroxyalkyle en C₁-C₄ ou hydroxyalcoxy en C₁-C₄,
acétylaminoalcoxy en C₁-C₄, mésylaminoalcoxy en C₁-C₄ ou carbamoylaminoalcoxy en C₁-C₄,
R₄ représente un atome d'hydrogène, d'halogène ou un radical alkyle en C₁-C₄.

4. Composition selon l'une quelconque des revendications précédentes dans laquelle la ou les bases d'oxydation choisies parmi les para-phénylènediamines sont présentes chacune en quantité comprise entre 0,001 à 10 % en poids environ du poids total de la composition tinctoriale.

5. Composition selon l'une quelconque des revendications précédentes dans laquelle le ou les méta-aminophénols sont choisis parmi les composés de formule (III) suivante et leurs sels d'addition : 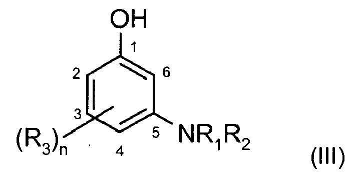 dans laquelle :
R₁ et R₂, identiques ou différents, représentent un atome d'hydrogène ; un radical alkyle ; un radical monohydroxyalkyle ; un radical polyhydroxyalkyle ; un radical monoaminoalkyle ;
R₁ et R₂ peuvent également former entre eux et avec l'atome d'azote auquel ils sont reliés un groupement cyclique contenant un ou plusieurs hétéroatomes, de 5 à 7 chaînons, saturé ou insaturé, non substitué ou substitué par un ou plusieurs radicaux choisis parmi les radicaux carboxy, carboxamido, hydroxyle, amino, mono ou dialkylamino, alkyle éventuellement substitué par un ou plusieurs radicaux hydroxyle, amino, mono ou dialkylamino ;
R₃ représentent, indépendamment les uns des autres, un atome d'halogène ; un radical alkyle ; un radical alcoxy ; un radical monohydroxyalkyle ; un radical polyhydroxyalkyle ; un radical monohydroxyalcoxy ; un radical polyhydroxyalcoxy ;
n est un entier compris entre 0 et 3.

6. Composition selon la revendication 5 dans laquelle R₁ et R₂ désignent indépendemment l'un de l'autre un atome d'hydrogène ou un radical mono ou polyhydroxyalkyle.

7. Composition selon l'une quelconque des revendications précédentes dans laquelle le ou les coupleurs choisis parmi les méta-aminophénols sont choisis parmi les méta-aminophénols chlorés.

8. Composition selon l'une quelconque des revendications précédentes dans laquelle le ou les coupleurs choisis parmi les méta-aminophénols sont chacun présents en quantité comprise entre 0,001 et 10 % en poids environ du poids total de la composition tinctoriale.

9. Composition selon l'une quelconque des revendications précédentes comprenant au moins une base d'oxydation additionnelle choisie parmi les bis-phénylalkylènediamines, les para-aminophénols, les bis-para-aminophénols, les ortho-aminophénols, les ortho-phénylènediamines, les bases hétérocycliques différentes de la 2,3-diamino-6,7-dihydro-1H,5H-pyrazolo[1,2-a]pyrazol-1-one et ses sels d'addition.

10. Composition selon l'une quelconque des revendications précédentes comprenant au moins un coupleur additionnel choisi parmi les méta-phénylènediamines, les méta-diphénols, les coupleurs naphtaléniques, les coupleurs hétérocycliques et leurs sels d'addition.

11. Composition selon l'une quelconque des revendications précédentes dans laquelle le ou les agents oxydants sont choisis parmi le peroxyde d'hydrogène, le peroxyde d'urée, les bromates de métaux alcalins, les persels, les peracides et les enzymes oxydases.

12. Composition selon l'une quelconque des revendications précédentes dans laquelle le ou les agents oxydants sont présents en quantité comprise entre 0,01 et 30 % en poids par rapport au poids total de la composition.

13. Procédé de teinture des fibres kératiniques dans lequel on applique sur les fibres la composition telle que définie à l'une quelconque des revendications 1 à 12 pendant un temps suffisant pour développer la coloration désirée, après quoi on rince, on lave éventuellement au shampooing, on rince à nouveau et on sèche.

14. Procédé selon la revendication 13 comportant une étape préliminaire consistant à stocker sous forme séparée, d'une part, une composition (A) comprenant, dans un milieu approprié pour la teinture, au moins une première base d'oxydation choisie parmi la 2,3-diamino-6,7-dihydro-1H,5H-pyrazolo[1,2-a]pyrazol-1-one et ses sels d'addition, au moins une deuxième base d'oxydation choisie parmi les para-phénylènediamines et au moins un coupleur choisi parmi les méta-aminophénols et d'autre part, une composition (B) renfermant, dans un milieu approprié pour la teinture, au moins un agent oxydant, puis à procéder à leur mélange au moment de l'emploi avant d'appliquer ce mélange sur les fibres kératiniques.

15. Dispositif à plusieurs compartiments dans lequel un premier compartiment contient une composition (A) telle que définie à la revendication 14 et un deuxième compartiment contient une composition (B) telle que définie à la revendication 14.

16. Utilisation pour la teinture d'oxydation des fibres kératiniques d'une composition telle que définie dans l'une quelconque des revendications 1 à 12.

## Claims

1. Composition for dyeing keratin fibres, comprising, in a suitable dyeing medium:
• at least one first oxidation base chosen from 2,3-diamino-6,7-dihydro-1H,5H-pyrazolo[1,2-a]pyrazol-1-one of formula (I) below, and the addition salts thereof: 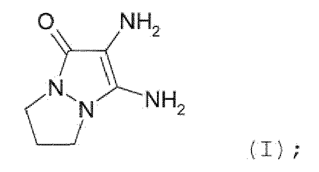
• at least one second oxidation base chosen from para-phenylenediamines;
• at least one coupler chosen from meta-aminophenols; and
• at least one oxidizing agent;
the pH of the composition ranging from 5.5 to 7.5.

2. Composition according to Claim 1, in which the oxidation base(s) chosen from 2,3-diamino-6,7-dihydro-1H,5H-pyrazolo[1,2-a]pyrazol-1-one of formula (I) and the addition salts thereof is (are) each present in an amount of between 0.001% and 10% by weight approximately relative to the total weight of the dye composition.

3. Composition according to Claim 1 or 2, in which the para-phenylenediamine(s) is (are) chosen from the compounds of formula (II) below, and the addition salts thereof: 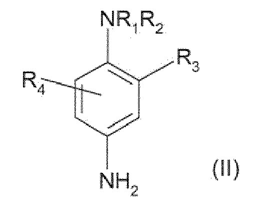 in which:
R₁ represents a hydrogen atom, a C₁-C₄ alkyl radical, a C₁-C₄ monohydroxyalkyl radical, a C₂-C₄ polyhydroxyalkyl radical, a (C₁-C₄) alkoxy (C₁-C₄) alkyl radical or a C₁-C₄ alkyl radical substituted with a nitrogenous, phenyl or 4'-aminophenyl group;
R₂ represents a hydrogen atom, a C₁-C₄ alkyl radical, a C₁-C₄ monohydroxyalkyl radical or C₂-C₄ polyhydroxyalkyl radical, a (C₁-C₄)alkoxy(C₁-C₄)alkyl radical or a C₁-C₄ alkyl radical substituted with a nitrogenous group;
R₁ and R₂ may also form, with the nitrogen atom that bears them, a 5- or 6-membered, saturated or unsaturated cyclic group containing one or more heteroatoms, optionally substituted with one or more alkyl, hydroxyl or ureido groups;
R₃ represents a hydrogen atom, a halogen atom, a C₁-C₄ alkyl radical, a sulfo radical, a carboxy radical, a C₁-C₄ monohydroxyalkyl radical or C₁-C₄ hydroxyalkoxy radical, an acetylamino (C₁-C₄) alkoxy radical, a mesylamino (C₁-C₄) alkoxy radical or a carbamoylamino (C₁-C₄)alkoxy radical,
R₄ represents a hydrogen or halogen atom or a C₁-C₄ alkyl radical.

4. Composition according to any one of the preceding claims, in which the oxidation base(s) chosen from para-phenylenediamines is (are) each present in an amount of between 0.001% and 10% by weight approximately relative to the total weight of the dye composition.

5. Composition according to ay one of the preceding claims, in which the meta-aminophenol(s) is (are) chosen from the compounds of formula (II) below, and the addition salts thereof: 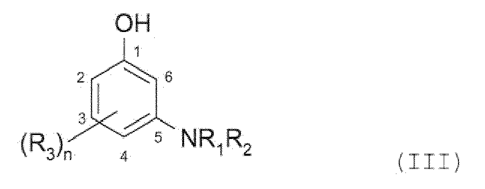 in which:
R₁ and R₂, which may be identical or different, represent a hydrogen atom; an alkyl radical; a monohydroxyalkyl radical; a polyhydroxyalkyl radical; a monoaminoalkyl radical;
R₁ and R₂ form, together with the nitrogen atom to which they are attached, a saturated or unsaturated, 5- to 7-membered cyclic group containing one or more heteroatoms, which may be unsubstituted or substituted with one or more radicals chosen from carboxyl, carboxamido, hydroxyl, amino, monoalkylamino and dialkylamino radicals, and alkyl radicals optionally substituted with one or more hydroxyl, amino, monoalkylamino or dialkylamino radicals;
R₃ represent, independently of each other, a halogen atom; an alkyl radical; an alkoxy radical; a monohydroxyalkyl radical; a polyhydroxyalkyl radical; a monohydroxyalkoxy radical; a polyhydroxyalkoxy radical;
n is an integer between 0 and 3.

6. Composition according to Claim 5, in which R₁ and R₂ denote, independently of each other, a hydrogen atom or a mono- or polyhydroxyalkyl radical.

7. Composition according to any one of the preceding claims, in which the coupler(s) chosen from meta-aminophenols is (are) chosen from chlorinated meta-aminophenols.

8. Composition according to any one of the preceding claims, in which the coupler(s) chosen from meta-aminophenols is (are) each present in an amount of between 0.001% and 10% by weight approximately relative to the total weight of the dye composition.

9. Composition according to any one of the preceding claims, comprising at least one additional oxidation base chosen from bis(phenyl)alkylenediamines, para-aminophenols, bis-para-aminophenols, ortho-aminophenols, ortho-phenylenediamines and heterocyclic bases other than 2,3-diamino-6,7-dihydro-1H, 5H-pyrazolo[1,2-a]pyrazol-1-one, and the addition salts thereof.

10. Composition according to any one of the preceding claims, comprising at least one additional coupler chosen from meta-phenylenediamines, meta-diphenols, naphthalene-based couplers and heterocyclic couplers, and the addition salts thereof.

11. Composition according to any one of the preceding claims, in which the oxidizing agent(s) is (are) chosen from hydrogen peroxide, urea peroxide, alkali metal bromates, persalts, peracids and oxidase enzymes.

12. Composition according to any one of the preceding claims, in which the oxidizing agent(s) is (are) present in an amount of between 0.01% and 30% by weight relative to the total weight of the composition.

13. Process for dyeing keratin fibres, in which the composition as defined in any one of Claims 1 to 12 is applied to the fibres for a time that is sufficient to develop the desired coloration, after which the fibres are rinsed, optionally washed with shampoo, rinsed again and dried.

14. Process according to Claim 13, comprising a preliminary step that consists in separately storing, on the one hand, a composition (A) comprising, in a suitable dyeing medium, at least one first oxidation base chosen from 2,3-diamino-6,7-dihydro-1H,5H-pyrazolo[1,2-a]pyrazol-1-one and the addition salts thereof, at least one second oxidation base chosen from para-phenylenediamines and at least one coupler chosen from meta-aminophenols, and, on the other hand, a composition (B) containing, in a suitable dyeing medium, at least one oxidizing agent, and then in mixing them together at the time of use before applying this mixture to the keratin fibres.

15. Multi-compartment device, in which a first compartment contains a composition (A) as defined in Claim 14 and a second compartment contains a composition (B) as defined in Claim 14.

16. Use, for the oxidation dyeing of keratin fibres, of a composition as defined in any one of Claims 1 to 12.

## Patentansprüche

1. Zusammensetzung zum Färben von Keratinfasern, umfassend in einem für die Färbung geeigneten Medium:
• mindestens eine erste Oxidationsbase, die unter 2,3-Diamino-6,7-dihydro-1H,5H-pyrazolo[1,2-a]-pyrazol-1-on der folgenden Formel (I) und Additionssalzen davon ausgewählt ist: 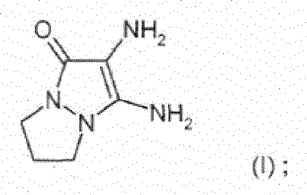
• mindestens eine zweite Oxidationsbase, die unter para-Phenylendiaminen ausgewählt ist;
• mindestens einen Kuppler, der unter meta-Aminophenolen ausgewählt ist; und
• mindestens ein Oxidationsmittel;
wobei der pH-Wert der Zusammensetzung im Bereich von 5,5 bis 7,5 liegt.

2. Zusammensetzung nach Anspruch 1, in der die Oxidationsbase bzw. die Oxidationsbasen, die unter 2,3-Diamino-6,7-dihydro-1H,5H-pyrazolo[1,2-a]-pyrazol-1-on der Formel (I) und Additionssalzen davon ausgewählt sind, jeweils in einer Menge zwischen etwa 0,001 und 10 Gew.-%, bezogen auf das Gesamtgewicht der Färbezusammensetzung, vorliegen.

3. Zusammensetzung nach Anspruch 1 oder 2, in der das para-Phenylendiamin bzw. die para-Phenylendiamine unter Verbindungen der folgenden Formel (II) und Additionssalzen davon ausgewählt sind: 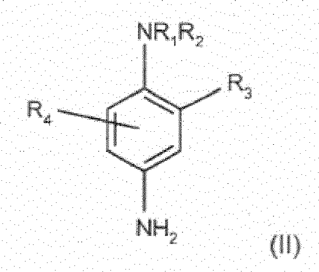 worin:
R₁ für ein Wasserstoffatom, einen C₁-C₄-Alkylrest, einen C₁-C₄-Monohydroxyalkylrest, einen C₂-C₄-Polyhydroxyalkylrest, einen (C₁-C₄) -Alkoxy- (C₁-C₄) - alkylrest, einen C₁-C₄-Alkylrest, der durch eine stickstoffhaltige Gruppe substituiert ist, einen Phenylrest oder einen 4'-Aminophenylrest steht;
R₂ für ein Wasserstoffatom, einen C₁-C₄-Alkylrest, einen C₁-C₄-Monohydroxyalkylrest, einen C₂-C₄-Polyhydroxyalkylrest, einen (C₁-C₄) -Alkoxy- (C₁-C₄) - alkylrest oder einen C₁-C₄-Alkylrest, der durch eine stickstoffhaltige Gruppe substituiert ist, steht;
R₁ und R₂ miteinander und mit dem Stickstoffatom, an das sie gebunden sind, eine 5- oder 6-gliedrige gesättigte oder ungesättigte cyclische Gruppe, die ein oder mehrere Heteroatome enthält und gegebenenfalls durch eine oder mehrere Alkyl-, Hydroxy- oder Ureidogruppen substituiert ist, bilden können;
R₃ für ein Wasserstoffatom, ein Halogenatom, einen C₁-C₄-Alkylrest, einen Sulforest, einen Carboxyrest, einen C₁-C₄-Monohydroxyalkylrest, einen C₁-C₄-Hydroxyalkoxyrest, einen Acetylamino-C₁-C₄-alkoxyrest, einen Mesylamino-C₁-C₄-alkoxyrest oder einen Carbamoylamino-C₁-C₄-alkoxyrest steht;
R₄ für ein Wasserstoffatom, ein Halogenatom oder einen C₁-C₄-Alkylrest steht.

4. Zusammensetzung nach einem der vorhergehenden Ansprüche, in der die Oxidationsbase bzw. die Oxidationsbasen, die unter para-Phenylendiaminen ausgewählt sind, jeweils in einer Menge zwischen etwa 0,001 und 10 Gew.-%, bezogen auf das Gesamtgewicht der Färbezusammensetzung, vorliegen.

5. Zusammensetzung nach einem der vorhergehenden Ansprüche, in der das meta-Aminophenol bzw. die meta-Aminophenole unter den Verbindungen der folgenden Formel (III) und Additionssalzen davon ausgewählt sind: 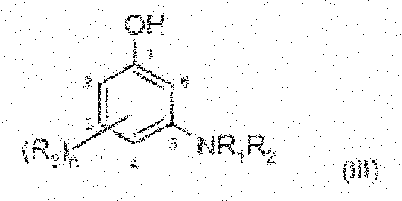 worin:
R₁ und R₂ gleich oder verschieden sind und für ein Wasserstoffatom, einen Alkylrest, einen Monohydroxyalkylrest, einen Polyhydroxyalkylrest oder einen Monoaminoalkylrest stehen;
R₁ und R₂ miteinander und mit dem Stickstoffatom, an das sie gebunden sind, eine 5- bis 7-gliedrige gesättigte oder ungesättigte cyclische Gruppe, die ein oder mehrere Heteroatome enthält und gegebenenfalls durch einen oder mehrere unter Carboxy-, Carboxamido-, Hydroxyl-, Amino-, Mono- oder Dialkylaminoresten und gegebenenfalls durch einen oder mehrere Hydroxyl-, Amino-, Mono- oder Dialkylaminoreste substituierten Alkylresten ausgewählte Reste substituiert ist, bilden können;
die Gruppen R₃ unabhängig voneinander für ein Halogenatom, einen Alkylrest, einen Alkoxyrest, einen Monohydroxyalkylrest, einen Polyhydroxyalkylrest, einen Monohydroxyalkoxyrest oder einen Polyhydroxyalkoxyrest stehen und
n für eine ganze Zahl zwischen 0 und 3 steht.

6. Zusammensetzung nach Anspruch 5, in der R₁ und R₂ unabhängig voneinander für ein Wasserstoffatom oder einen Mono- oder Polyhydroxyalkylrest stehen.

7. Zusammensetzung nach einem der vorhergehenden Ansprüche, in der der bzw. die unter den meta-Aminophenolen ausgewählte(n) Kuppler unter chlorierten meta-Aminophenolen ausgewählt sind.

8. Zusammensetzung nach einem der vorhergehenden Ansprüche, in der der bzw. die unter den meta-Aminophenolen ausgewählte(n) Kuppler jeweils in einer Menge zwischen etwa 0,001 und 10 Gew.-%, bezogen auf das Gesamtgewicht der Färbezusammensetzung, vorliegen.

9. Zusammensetzung nach einem der vorhergehenden Ansprüche, umfassend mindestens eine zusätzliche Oxidationsbase, die unter Bisphenylalkylendiaminen, para-Aminophenolen, Bis-para-aminophenolen, ortho-Aminophenolen, ortho-Phenylendiaminen, heterocyclischen Basen, die von 2,3-Diamino-6,7-dihydro-1H,5H-pyrazolo[1,2-a]pyrazol-1-on verschieden sind, sowie Additionssalzen davon ausgewählt ist.

10. Zusammensetzung nach einem der vorhergehenden Ansprüche, umfassend mindestens einen zusätzlichen Kuppler, der unter meta-Phenylendiaminen, meta-Diphenolen, Kupplern auf Naphthalinbasis, heterocyclischen Kupplern sowie Additionssalzen davon ausgewählt ist.

11. Zusammensetzung nach einem der vorhergehenden Ansprüche, in der das Oxidationsmittel bzw. die Oxidationsmittel unter Wasserstoffperoxid, Harnstoffperoxid, Alkalimetallbromaten, Persalzen, Persäuren und Oxidaseenzymen ausgewählt sind.

12. Zusammensetzung nach einem der vorhergehenden Ansprüche, in der das Oxidationsmittel bzw. die Oxidationsmittel in einer Menge zwischen 0,01 und 30 Gew.-%, bezogen auf das Gesamtgewicht der Zusammensetzung, vorliegen.

13. Verfahren zum Färben von Keratinfasern, bei dem man auf die Fasern die Zusammensetzung gemäß einem der Ansprüche 1 bis 12 über einen zur Entwicklung der gewünschten Färbung ausreichenden Zeitraum aufbringt und dann spült, gegebenenfalls mit Shampoo wäscht, erneut spült und trocknet.

14. Verfahren nach Anspruch 13 mit einem vorgeschalteten Schritt, bestehend aus der getrennten Aufbewahrung einerseits einer Zusammensetzung (A), die in einem für die Färbung geeigneten Medium mindestens eine erste Oxidationsbase, die unter 2,3-Diamino-6,7-dihydro-1H,5H-pyrazolo[1,2-a]-pyrazol-1-on und Additionssalzen davon ausgewählt ist, mindestens eine zweite Oxidationsbase, die unter para-Phenylendiaminen ausgewählt ist, und mindestens einen Kuppler, der unter meta-Aminophenolen ausgewählt ist, umfasst, und andererseits einer Zusammensetzung (B), die in einem für die Färbung geeigneten Medium mindestens ein Oxidationsmittel enthält, und nachfolgendem Mischen davon zum Zeitpunkt der Anwendung vor dem Aufbringen dieser Mischung auf die Keratinfasern.

15. Vorrichtung mit mehreren Kompartimenten, in der ein erstes Kompartiment eine Zusammensetzung (A) gemäß Anspruch 14 enthält und ein zweites Kompartiment eine Zusammensetzung (B) gemäß Anspruch 14 enthält.

16. Verwendung einer Zusammensetzung gemäß einem der Ansprüche 1 bis 12 zum Oxidationsfärben von Keratinfasern.
